Europäisches Patentamt

European Patent Office

Office européen des brevets

(11) Publication number: **0 097 439**
**B1**

## EUROPEAN PATENT SPECIFICATION

(45) Date of publication of patent specification: **15.03.89**

(51) Int. Cl.⁴: **C 07 C 177/00,** A 61 K 31/557

(21) Application number: **83303141.2**

(22) Date of filing: **01.06.83**

(54) **A crystalline prostaglandin salt.**

(30) Priority: **18.06.82 US 389730**

(43) Date of publication of application:
**04.01.84 Bulletin 84/01**

(45) Publication of the grant of the patent:
**15.03.89 Bulletin 89/11**

(84) Designated Contracting States:
**BE CH DE FR GB IT LI NL SE**

(56) References cited:
**EP-A-0 045 661**
**US-A-4 060 534**

(73) Proprietor: **THE UPJOHN COMPANY**
**301 Henrietta Street**
**Kalamazoo, Michigan 49001 (US)**

(72) Inventor: **Bundy, Gordon Leonard**
**c/o The Upjohn Company 301 Henrietta Street**
**Kalamazoo Michigan 49001 (US)**

(74) Representative: **Perry, Robert Edward et al**
**GILL JENNINGS & EVERY 53-64 Chancery Lane**
**London WC2A 1HN (GB)**

EP 0 097 439 B1

**Description**

The present invention relates to a novel crystalline salt of 9-deoxo-16,16-dimethyl-9-methylene-PGE (herein "the free acid").

The free acid (a viscous oil) is disclosed in US—A—3950363 as a useful gastric antisecretory agent, as are lower alkyl esters thereof. US—A—4060534 discloses the free acid and various salt forms thereof, e.g. the tris(hydroxymethyl)aminomethane ("THAM") salt and also pharmacologically-acceptable salts with cations of pharmacologically-acceptable metals, especially alkali metals, e.g. lithium, sodium and potassium, and alkaline earth metals, e.g. magnesium and calcium.

Prostaglandins are known generally to be oily materials, which complicates their handling and formulation for pharmaceutical purposes. Crystalline forms of certain prostaglandins are, however, known to exist. For example, $PGF_{2\alpha}$ is an oily material which is difficult to purify and formulate pharmaceutically. However, US—A—3657327 describes a free-flowing crystalline THAM salt of $PGF_{2\alpha}$. Other solid $PGF_{2\alpha}$ salts, including the L-arginine salt, are described in US—A—4005133.

For relatively unstable prostaglandin free acids, the preparation of crystalline salts thereof is known to increase stability markedly. For example, US—A—4338325 describes the crystalline sodium salt of prostacyclin or $PGI_2$.

A novel salt according to the present invention is 9-deoxo-16,16-dimethyl-9-methylene-$PGE_2$ potassium salt, m.p. 130—132°C.

By contrast with the free acid, the novel salt is pharmaceutically elegant. For example, it is easily and simply prepared using conventional crystallisation techniques. Its crystallinity facilitates handling and chemical analysis, and improves chemical stability. Moreover, it is non-hygroscopic, a characteristic of importance and significance in handling and formulation for pharmaceutical purposes.

The novel salt is simply formulated, especially in suppositories, by mixing it with conventional suppository bases; drug release rates therefrom when administered to a patient or animal bioequivalently provide the useful pharmacological effects of the free acid. The biological profile of the novel salt is essentially equivalent to that of the free acid; the salt is thus useful for the known pharmacological purposes for which the free acid is employed, and provides an especially useful and unexpectedly improved means whereby the free acid is employed for pharmaceutical purposes.

The present invention may be more completely understood by the operation of the following examples:

## Example 1

All solvents used in this example are degassed with nitrogen using a gas dispersion tube for 15 min prior to use. To a stirred solution of 4.82 g of 9-deoxo-16,16-dimethyl-9-methylene-$PGE_2$ in 50 ml of methanol is added a solution of 880 mg of potassium carbonate in 15 ml of water. The reaction mixture is then allowed to stand for 5 min at 25°C and then concentrated under reduced pressure at 35°C. Eight 250 ml portions of anhydrous tetrahydrofuran are added and evaporated to insure complete removal of water. The residue, a pale yellow foam, is dissolved in 15 ml of methanol and added dropwise under a nitrogen atmosphere with vigorous stirring to one liter of diethyl ether. The initial cloudy suspension yields a gum-like residue on the walls of the reaction vessel after about 1 hr. Continued vigorous stirring for 24 hr leads to gradual crystallization and resuspension as a uniform, easily filterable solid. This suspension is then filtered using a medium porosity sintered glass funnel. The solids are washed with 15 ml of fresh diethyl ether at 25°C. After drying for 2 hr in a vacuum desiccator at 25°C, a colorless free-flowing crystalline solid, 5.13 g, is obtained, pure crystalline 9-deoxo-16,16-dimethyl-9-methylene-$PGE_2$ potassium salt. IR absorptions ($cm^{-1}$) are observed at 3134, 1652, 1572, 1467, 1392, 1337, 1364, 1344, 1102, 1024, 974, and 883. Water content is 0.16% after 5 days at ambient humidity. Melting range is 130—132°C.

## Example 2

9-Deoxo-16,16-dimethyl-9-methylene-$PGE_2$ (57.03 g) is dissolved in methanol. Thereafter the resulting mixture is stirred in an ice bath and a solution of 10.11 g of potassium carbonate in water is added. The water and methanol are then removed by concentration under reduced pressure at 35°C and thereafter 500 ml of dry tetrahydrofuran is added and evaporated under reduced pressure. Five additional 500 ml portions of dry tetrahydrofuran are similarly added and evaporated. Thereafter a pale yellow foam is obtained and dissolved in 75 ml of methanol. This methanolic solution is then added dropwise to 4 liters of anhydrous diethyl ether and the potassium salt formed an oily or gummy mixture on the walls of the reaction vessels. After crystallization, induced by stirring for 4 hr, the product is scraped from the walls of the reaction vessel and the resulting suspension is stirred vigorously for 64 hr. After filtration and washing of the solids with 500 ml of diethyl ether, pure crystalline product is then dried over nitrogen and desiccated under reduced pressure for 16 hr. Pure crystalline 9-deoxo-16,16-dimethyl-9-methylene-$PGE_2$ potassium salt (59.12 g) is obtained.

## Example 3

9-Deoxo-16,16-dimethyl-9-methylene-$PGE_2$, methyl ester (110 g) in 169 ml of methanol is added to 149 ml of 1N aqueous potassium hydroxide to yield a cloudy mixture. This mixture is then heated to 65°C for 15 min and diluted with 2 liters of tetrahydrofuran. Concentration on a rotary evaporator at 41—45°C is undertaken concomitently with the addition of additional tetrahydrofuran until a total of 15.2 liters of tetrahydrofuran has been removed. The resulting residue is then dissolved in 125 ml of warm methanol and 1,250 ml of anhydrous tetrahydrofuran. The resulting mixture is then stirred and 1 liter of hexane is added. Seed crystals are then added (the product of Example 1 or Example 2) and an additional 375 ml of hexane is added dropwise. The resulting mixture is then stirred for 15 hrs, while adding dropwise an additional 500 ml of hexane. After stirring for 2 hrs and filtering, the resulting crystalline 9-deoxo-16,16-di-methyl-9-methylene PGE$_2$ potassium salt is washed in 25% tetrahydrofuran in hexane to yield 61.6 g of pure crystalline product.

Optionally, recrystallization from methanol, tetrahydrofuran and hexane (1:10:30) yields pure title product. Also, additional pure crystalline product can be obtained by concentration of mother liquors to an oil followed by recrystallization from methanol, tetrahydrofuran and hexane (1:10:18).

Example 4

A. Midtrimester pregnant female Rhesus monkeys (*Macaca mulatta*) are used. (The first day of menstrual bleeding is designated Day 1 of the menstrual cycle; the first day a female is placed with a male for breeding is designated Day 1 of pregnancy. Pregnancy is confirmed by abdominal palpation of the uterus.)

B. A fluid-filled open-ended polyethylene tubing (PE10, Clay Adams) inserted transabdominally into the amniotic sac is used to monitor uterine muscle activity in pregnant animals. The polyethylene tubing is attached to transducers which are in turn connected to a polygraph. Animals are anesthetized when recordings are made. Animals are fasted overnight prior to use.

Blood samples are collected into heparinized syringes at specified times following insertion of a suppository in pregnant or non-pregnant monkeys. Plasma is extracted and assayed for 9-deoxo-16,16-di-methyl-9-methylene-PGE$_2$ content by radioimmunoassay.

C. Six suppository lots are prepared (4), each containing 9-deoxo-16,16-dimethyl-9-methylene-PGE$_2$ or its crystalline potassium salt (Examples 1 and 2). Two lots are made with Witepsol H15 alone as the suppository base, two with Witepsol H15 wax plus 2% colloidal silicon dioxide (CSD) and two with Witepsol H15 plus 2% CSD plus 10% triacetin. Suppositories are prepared using Witepsol H15 suppository wax. Free acid or potassium salt is dispersed in the hot wax using a homogenizer. Two percent colloidal silicon dioxide (CSD) is mixed in by further homogenization. In the third formulation, triacetin (10%) is added before the CSD and then homogenized for one minute. Suppositories are formed in 800 mg aluminum molds and are stored at 4°C until used.

D. The plasma levels of 9-deoxo-16,16-dimethyl-9-methylene-PGE$_2$ in monkeys treated with 6 formulations containing either 9-deoxo-16,16-dimethyl-9-methylene-PGE$_2$ free acid or potassium salt are shown in Tables 1—6. In general, higher blood levels are measured in pregnant Rhesus monkeys than in non-pregnant monkeys. Similar plasma drug levels are reached in monkeys treated with either free acid or potassium salt.

The uterine activity of pregnant Rhesus monkeys treated with these formulations in all cases begins by increased uterine tone 2—3 minutes following insertion of the suppository into the vagina. After 8—10 min the pattern changes into high amplitude frequent contractions lasting the length of the recording period.

E. Examination of the drug plasma levels and uterine activity in Rhesus monkeys following treatment with free acid and potassium salt at 30 mg (acid equivalents) per dose in three suppository formulations indicates that these two compounds are equally bioavailable.

Table 1

Plasma Levels in Rhesus Monkeys Treated with
30 mg Free Acid in H15 Suppositories

| Hours | Drug Levels (ng/ml) | | |
|---|---|---|---|
| | Monkey #1 | Monkey #2 | Monkey #3[a] |
| 0 | ND | ND | ND |
| 0.25 | 5.14 | 0.61 | 142.16 |
| .5 | 1.69 | ND | 63.60 |
| 1.0 | 4.39 | ND | 93.90 |
| 1.5 | 7.89 | 3.35 | 66.84 |
| 2.0 | 4.16 | 5.56 | 60.76 |
| 3.0 | 0.23 | 12.99 | 56.09 |
| 4.0 | 7.43 | 14.70 | 35.66 |
| 6.0 | 10.08 | 17.26 | 13.67 |
| 24.0 | 7.68 | 4.97 | 5.67 |

Table 2

Plasma Levels in Rhesus Monkeys Treated with
30 mg Potassium Salt in H15 Suppositories

| Hours | Drug Levels (ng/ml) | | |
|---|---|---|---|
| | Monkey #4 | Monkey #5 | Monkey #6[a] |
| 0 | ND | ND | ND |
| 0.25 | 127.99 | 10.88 | 76.56 |
| 0.5 | 3.72 | ND | 68.37 |
| 1 | ND | 1.42 | 29.59 |
| 1.5 | 0.41 | 5.91 | 77.27 |
| 2 | 0.86 | 11.54 | 28.39 |
| 3 | 7.52 | 14.18 | 41.68 |
| 4 | 8.39 | 22.91 | 42.76 |
| 6 | 17.88 | 25.04 | 20.20 |
| 24 | 21.69 | 16.24 | 7.77 |

[a]Pregnant animal with uterine recording

Table 3

Plasma Levels in Rhesus Monkeys Treated with
30 mg Free Acid in H15 Plus 2% CSD Suppositories

| Hours | Drug Levels (ng/ml) | | |
|---|---|---|---|
| | Monkey #7 | Monkey #8 | Monkey #9[a] |
| 0 | ND | ND | ND |
| 0.25 | 39.17 | 51.44 | 60.72 |
| 0.5 | 44.46 | 133.94 | 52.17 |
| 1.0 | 63.93 | 88.18 | 63.06 |
| 1.5 | 42.17 | 61.10 | 62.77 |
| 2 | 36.95 | 78.35 | 62.85 |
| 3 | 16.94 | 29.05 | 40.16 |
| 4 | 13.25 | 12.36 | 57.77 |
| 6 | 6.50 | 2.49 | 32.03 |
| 24 | 4.01 | 15.72 | 20.98 |

Table 4

Plasma Levels in Rhesus Monkeys Treated with
30 mg Potassium Salt in H15 Plus 2% CSD
Suppositories

| Hours | Drug Levels (ng/ml) | | |
|-------|-----------|-----------|------------|
| | Monkey #10 | Monkey #11 | Monkey #12[a] |
| 0 | ND | ND | ND |
| 0.25 | 20.41 | 19.84 | 103.77 |
| .5 | 2.39 | 13.98 | 55.66 |
| 1 | 1.50 | 6.61 | 40.86 |
| 1.5 | 1.70 | 8.13 | 91.09 |
| 2 | 3.57 | 9.80 | 66.54 |
| 3 | 12.38 | 9.48 | 51.46 |
| 4 | 20.66 | 18.66 | 36.12 |
| 6 | 53.76 | 28.67 | 22.51 |
| 24 | 15.97 | 23.37 | 6.04 |

[a]Pregnant animal with uterine recording

Table 5

Plasma Levels in Rhesus Monkeys Treated with
30 mg Free Acid in H15
Plus 2% CSD + 10% Triacetin Suppositories

| Hours | Drug Levels (ng/ml) | | |
|-------|-----------|-----------|------------|
| | Monkey #13 | Monkey #14 | Monkey #15[a] |
| 0 | ND | ND | ND |
| 0.25 | 12.56 | 18.80 | 118.62 |
| 0.5 | 9.25 | 35.96 | 154.08 |
| 1 | 2.80 | 25.06 | 154. |
| 1.5 | 3.09 | 32.01 | 26.82 |
| 2 | 5.46 | 28.73 | 44.83 |
| 3 | 15.10 | 20.19 | 65.87 |
| 4 | 22.54 | 16.61 | 22.05 |
| 6 | 23.81 | 16.25 | 13.00 |
| 24 | 25.96 | 12.89 | 2.76 |

5

Table 6

Plasma Levels in Rhesus Monkeys Treated with
30 mg Potassium Salt in H15
Plus 2% CSD + 10% Triacetin Suppositories

| Hours | Drug Levels (ng/ml) | | |
|---|---|---|---|
| | Monkey #16 | Monkey #17 | Monkey #18 |
| 0 | ND | ND | ND |
| 0.25 | 7.45 | 7.98 | 56.78 |
| 0.5 | 7.00 | 2.15 | 207.06 |
| 1 | 12.62 | 1.27 | 64.39 |
| 1.5 | 19.88 | 1.86 | 38.30 |
| 2 | 40.18 | 1.75 | 87.17 |
| 3 | 45.45 | 20.11 | 47.84 |
| 4 | 31.38 | 3.13 | 35.46 |
| 6 | 34.47 | 6.97 | 33.85 |
| 24 | 14.21 | 21.94 | 8.31 |

**Claim**

Crystalline 9-deoxo-16,16-dimethyl-9-methylene-$PGE_2$ potassium salt, m.p. 130—132°C.

**Patentanspruch**

Kristallines 9-Deoxo-16,16-Dimethyl-9-Methylen-$PGE_2$-Kaliumsalz, Schmelzpunkt 130—132°C.

**Revendication**

Sel de potassium de 9-désoxo-16,16-diméthyl-9-méthylène-$PGE_2$ cristallin, P.F. 130—132°C.